# EUROPÄISCHE PATENTSCHRIFT

(11) **EP 0 960 933 B1**
(45) Veröffentlichungstag und Bekanntmachung des Hinweises auf die Patenterteilung: **25.08.2004**
(21) Anmeldenummer: 99109415.2
(22) Anmeldetag: 11.05.1999
(51) Int. Cl.: C12M 3/00, C12N 15/89

(54) **Verfahren und Vorrichtung zur Messung einer Zustandsgrösse**
Measuring apparatus and process
Appareil et procédé de mesure

(30) Priorität: 27.05.1998 DE 19823655; 23.06.1998 DE 29811066 U
(43) Veröffentlichungstag der Anmeldung: 01.12.1999
(73) Patentinhaber: Micronas GmbH, 79108 Freiburg (DE)
(72) Erfinder: Baumann, Werner, Dr., 77815 Bühl (DE); Ehret, Ralf, Dr., 79291 Merdingen (DE); Lehman, Mirko, Dipl.-Phys., 79117 Freiburg (DE); Igel, Günter, Dipl.-Ing., 79331 Teningen (DE); Gahle, Jürgen, Dr. Ing., 79312 Emmendingen (DE); Wolf, Bernhard, Prof. Dr., 79252 Stegen (DE); Sieben, Ulrich, Dr. Dipl-Phys., 79276 Reute (DE); Freund, Ingo, Dipl.-Ing., 79235 Vogtsburg-Oberrotweil (DE); Brischwein, Martin, Dr., 97424 Schweinfurt (DE)
(74) Vertreter: Patentanwälte Westphal, Mussgnug & Partner

(56) Entgegenhaltungen:
- EP-A- 0 292 899
- EP-A- 0 539 660
- WO-A-97/17426
- DE-A- 4 004 198
- DE-A- 4 031 138
- DE-A- 4 400 955
- US-A- 5 262 128
- US-A- 5 457 041
- DATABASE WPI Section Ch, Week 8815 Derwent Publications Ltd., London, GB; Class D16, AN 88-102472 XP002115883 & JP 63 052871 A (SHIMADZU SEISAKUSHO KK), 7. März 1988 (1988-03-07)

## Beschreibung

Die Erfindung betrifft ein Verfahren zum Messen mindestens einer Zustandsgröße einer in einem Nährmedium befindlichen, adhärent an einem Auflagebereich angelagerten biologischen Zelle, wobei in die Zellmembran der Zelle zum Messen der Zustandsgröße wenigstens eine Öffnung eingebracht wird. Die Erfindung bezieht sich ferner auf eine Vorrichtung zur Messung mindestens einer Zustandsgröße wenigstens einer in einem Nährmedium befindlichen biologischen Zelle, mit einem Objektträger, der einen Auflagebereich hat, an dem die Zelle adhärent anlagerbar ist, mit wenigstens einer mit der im Inneren der Zelle befindlichen Zellflüssigkeit in Kontakt bringbaren Meßsonde zum Messen der Zustandsgröße, wobei die Meßsonde mit einem Meßverstärker verbindbar oder verbunden ist.

Aus dem Buch Physologie des Menschen, Schmidt; Thews (Herausgeber), 23. Aufl. (1987), Seite 20-21 kennt man bereits eine Vorrichtung der eingangs genannten Art, die eine mit einer Absaugvorrichtung verbundene Hohlnadel mit einer Innenhöhlung aufweist, die am freien Ende der Hohlnadel eine Öffnung hat. In der Innenhöhlung der Hohlnadel ist eine Meßsonde zum Messen des Zellpotentials der Zelle angeordnet. Bei dieser, nach dem sogenannten Patch-Clamp-Verfahren arbeitenden Vorrichtung wird die Hohlnadel zum Inkontaktbringen der Meßelektrode mit der Zellflüssigkeit der Zelle mit der am freien Ende der Hohlnadel befindlichen Öffnung außenseitig an der Zellmembran angesetzt, um dann mittels der Absaugvorrichtung einen Unterdruck in der Innenhöhlung der Hohlnadel zu erzeugen. Durch diesen Unterdruck wird ein vor der Öffnung der Hohlnadel befindliches Zellmembranstück aus dem Membranverband herausgerissen. Durch die dabei entstehende Öffnung in der Zellmembran geraten die in der Zellflüssigkeit befindlichen Ionen in einen in der Hohlnadel befindlichen Elektrolyten und von dort zu der Meßsonde. Eine Referenzelektrode dient zum Ermitteln eines Bezugspotentials.

Das vorbekannte Verfahren und die entsprechende Vorrichtung haben den Nachteil, daß zum Positionieren der Hohlnadel an der Zelle ein Mikromanipulator erforderlich ist. Dadurch ergibt sich eine vergleichsweise komplizierte und teure Vorrichtung. Außerdem wird die Zugänglichkeit der auf dem Objektträger befindlichen Zellen durch den Mikromanipulator stark eingeschränkt. Das Verfahren und die Vorrichtung eignen sich deshalb nur für eine Untersuchung einzelner oder allenfalls für eine gleichzeitige Untersuchung einer kleinen Anzahl auf dem Objektträger befindlicher Zellen.

Aus US 4 461 304 ist ferner eine Vorrichtung bekannt, die eine nadelförmige Spitze zum Einbringen einer Öffnung in eine Zellmembran hat. An der Spitze sind eine Vielzahl von Sensoren für neurophysiologische Untersuchungen angeordnet. Auch bei dieser Vorrichtung ist zum Positionieren der Spitze an der Zelle ein Mikromanipulator erforderlich.

Aus EP 0 689 051 A2, DE 197 12 309 A1, DE 195 29 371 C2 und EP 0 585 933 A2 kennt man auch bereits Vorrichtungen zur Messung eines Zellpotentials, die einen Objektträger aufweisen, der eine Vielzahl von in Matrixform angeordneten Mikroelektroden hat, die mit der Außenseite der Zellmembran einer zu untersuchenden Zelle in Verbindung bringbar sind. Diese Vorrichtungen ermöglichen jedoch nur eine extrazelluläre Messung des Zellpotentials, da in die Zellmembran keine Öffnung eingebracht wird.

Aus DE 195 36 389 A1 und DE 195 36 384 A1 sind auch bereits Verfahren zum Messen einer Zustandsgröße bekannt, bei denen eine Biokomponente kontaktiert wird. Auch bei diesem Verfahren wird in die Biokomponente eine Öffnung nicht eingebracht.

In DE 38 16 458 A1 ist ferner eine Mikroelektrode beschrieben, die zu potentiometrischen oder amperometrischen Messung im biochemischen und medizinischen Bereich eingesetzt werden kann.

Die DE 44 22 049 C2 offenbart ein Ultra-Mikroelektroden-Array für chemische und biochemische Analysen, das auf einem Substrat mehrere Pyramiden oder kegelförmige Elektrodenspitzen aufweist. Nach Angabe der Patentschrift läßt sich das Ultra-Mikroelektroden-Array in Elektrodenkörpern für die Sauerstoffmessung nach Clark einsetzen.

Aus US 5 173 158 A ist außerdem ein gattungsfremdes Verfahren zum Generieren neuer Zellen bekannt, bei dem in einem Fluid befindliche Zellen einer ersten Art mittels Unterdruck oder hydrostatischem Druck derart an einem Auflagebereich einer porösen Schicht angelagert werden, daß die Zellen mit einem Teilbereich in die Poren der porösen Schicht eingreifen. Die poröse Schicht mit den Zellen ist zwischen an das Fluid angrenzenden Elektrodenplatten angeordnet, an die eine elektrische Spannung angelegt wird, welche die Zellmembran der Zellen in dem in die Poren eingreifenden Teilbereich öffnet. Danach werden durch diese Öffnungen hindurch Zellen einer zweiten Art in das innere der Zellen der ersten Art gebracht.

Es besteht deshalb die Aufgabe, ein Verfahren und eine Vorrichtung der eingangs genannten Art zu schaffen, die ein einfaches Messen einer Zustandsgröße der Zelle ermöglichen. Insbesondere soll ein aufwendiges manuelles Positionieren einer Hohlnadel an der zu untersuchenden Zelle vermieden werden.

Die Lösung dieser Aufgabe besteht bezüglich des Verfahrens darin, daß die Öffnung innerhalb des Auflagebereichs der Zelle und mit Abstand von dessen Rand in die Zellmembran eingebracht wird und daß durch diese Öffnung hindurch die Zustandsgröße gemessen wird.

Dadurch ist es möglich, ein für das Einbringen der Zellmembran-Öffnung verwendetes Porationsmittel oder ein Porationswerkzeug in dem Auflagebereich der Zelle an dem Objektträger anzuordnen, so daß die Zelle beim Anlagern an dem Auflagebereich gleichzeitig auch an dem Porationsmittel oder dem Porationswerkzeug positioniert ist. Dadurch kann ein aufwendiges manuelles Positionieren eines Porationswerkzeuges entfallen. Da die Öffnung innerhalb des Auflagebereichs der Zelle und mit Abstand vom Rand des Auflagebereichs in die Zellmembran eingebracht wird, dichtet der die Öffnung umgrenzende, adhärent an dem Auflagebereich anhaftende Membranbereich der Zelle die Öffnung gegen die Nährflüssigkeit ab. Die im Inneren der Zelle befindliche Zellflüssigkeit ist dadurch elektrisch weitgehend gegen die Nährflüssigkeit isoliert. Die gemessene Zustandsgröße der Zelle kann beispielsweise eine Ionenkonzentration, ein Gasgehalt, eine Temperatur oder eine beliebige andere physikalische, chemische oder biologische Eigenschaft einer Zelle sein.

Zum Messen des Zellpotentials der Zelle kann durch die in die Zellmembran der Zelle eingebrachte Öffnung hindurch die elektrische Spannung zwischen der Zellflüssigkeit und dem Nährmedium gemessen werden. Mit diesem Verfahren können beispielsweise die zwischen Nervenzellen übertragenen elektrischen Signale untersucht werden. Mit dem Verfahren können elektrische Gleichspannungs- und/oder Wechselspannungspotentiale, insbesondere zeitlich schnell veränderliche Potentiale gemessen werden.

Bei einer besonders vorteilhaften Ausführungsform der Erfindung wird die Öffnung mittels Elektroporation in die Zellmembran eingebracht. Bei der Durchführung des Verfahrens kann beispielsweise eine Elektroporations-Elektrode in dem Auflagebereich für die Zelle angeordnet werden, an der sich die Zelle adhärent anlagert. Zum Einbringen der Öffnung in die Zellmembran braucht dann nur noch eine elektrische Spannung zwischen der Elektroporations-Elektrode und dem Nährmedium angelegt zu werden, die einen elektrischen Stromfluß bewirkt, der die Zellmembran öffnet. Nach dem Abschalten der Elektroporationsspannung kann durch die Öffnung der Zellmembran hindurch die elektrische Spannung zwischen der Zellflüssigkeit und dem Nährmedium gemessen werden. Dabei kann gegebenenfalls die zur Elektroporation verwendete Elektrode auch zur Messung des Zellpotentials verwendet werden, so daß der Elektrode eine Doppelfunktion zukommt.

Bei einer anderen Ausführungsform der Erfindung wird zum Einbringen der Öffnung in die Zellmembran auf einen Teilbereich der Zellmembran wenigstens ein mechanischer Impuls ausgeübt. Dabei löst sich dieser Teilbereich der Zellmembran aus dem Membranverbund heraus. Gegebenenfalls kann auch eine Impulsfolge mit mehreren Einzelimpulsen zur Anwendung kommen.

Besonders vorteilhaft ist, wenn die Öffnung mittels Ultraschall in die Zellmembran eingebracht wird. Dabei ist es sogar möglich, daß der Ultraschall auf den zu öffnenden Bereich der Zellmembran fokussiert wird und/oder daß mehrere Ultraschallwellen derart überlagert werden, daß sich ihre Schwingungen in dem zu öffnenden Bereich der Zellmembran zu einer Schwingung mit erhöhter Amplitude überlagern. Die Zellmembran kann dadurch berührungslos geöffnet werden.

Eine berührungslose Öffnung der Zellmembran kann aber auch in der Weise erfolgen, daß ein Teilbereich der Zellmembran mit energiereicher Strahlung, insbesondere mit Laserstrahlung bestrahlt wird. Dabei wird die Wellenlänge der Strahlung vorzugsweise so gewählt, daß die Zellmembran die Strahlung gut absorbiert. Zweckmäßigerweise wird die Strahlung an dem Auflagebereich der Zelle in diese eingekoppelt. Gegebenenfalls kann ein Laserstrahl aber auch außerhalb des Auflagebereichs in die Zelle eingekoppelt werden, indem in einen dort befindlichen Membranbereich zunächst eine kleine Einkoppelöffnung eingebracht wird, durch die der Laserstrahl anschließend durch das Innere der Zelle hindurch auf einen im Auflagebereich der Zelle befindlichen Membranbereich projiziert wird, um dort durch Verschwenken des Laserstrahls um die Einkoppelöffnung einen Teilbereich der Membran aus dem Membranverbund herauszuschneiden.

Bei einer anderen Ausführungsform des Verfahrens wird die Öffnung durch Einwirkung einer chemischen Substanz in die Zellmembran eingebracht. Als Portionsmittel kann beispielsweise ein Perforin oder Triton® verwendet werden.

Besonders vorteilhaft ist, wenn eine elektrisch und/oder chemisch und/oder durch Strahlung aktivierbare chemische Substanz verwendet wird und wenn diese Substanz zum Einbringen der Öffnung in die Zellmembran durch Einwirkung von Strahlung und/oder eines elektrischen Feldes aktiviert wird. Die Substanz wird also durch Energiezufuhr aktiviert. Dabei können zum Beispiel freie Radikale erzeugt werden, welche den zu öffnenden Teilbereich der Zellmembran zerstören. Im inaktiven Zustand verhält sich die Substanz gegenüber der Zelle weitgehend neutral, so daß sie das Anlagern der Zelle an dem Auflagebereich praktisch nicht beeinflußt. Es kann auch eine chemische Substanz verwendet werden, die durch Zugabe einer weiteren Substanz chemisch aktiviert wird.

Eine andere Ausführungsform des Verfahrens sieht vor, daß ein zu öffnender Teilbereich der Zellmembran durch Beaufschlagung mit einem Unterdruck und/oder einem Überdruck aus dem Membranverband herausgelöst wird. Dazu kann beispielsweise in einem den Auflagebereich aufweisenden Objektträger innerhalb des Auflagebereichs eine kleine Öffnung vorgesehen sein, durch welche die Zelle so stark angesaugt wird, daß der vor der Öffnung befindliche Membranbereich aus dem Membranverbund herausgerissen wird. Zum Öffnen der Zellmembran kann durch die Öffnung hindurch aber auch ein Überdruckimpuls auf einen Membranbereich der Zelle ausgeübt werden.

Vorteilhaft ist, daß die Zelle durch eine Saugkraft an dem Auflagebereich fixiert wird. Dadurch kann das Anhaften der Zelle an dem Auflagebereich eines Objektträgers verbessert werden. Die Saugkraft ist dabei so bemessen, daß die Zellmembran durch die Saugkraft mechanisch nicht beschädigt wird.

Vorteilhaft ist, wenn nach dem Einbringen der Öffnung in die Zellmembran durch die Öffnung hindurch Zellflüssikeit aus der Zelle entnomen und untersucht wird. Dadurch ist es möglich, zusätzliche Zellgrößen, die im Inneren der Zelle nur schlecht meßbar sind, zu detektieren. Somit können zusätzliche Informationen über die Physiologie der Zelle gewonnen werden.

Besonders vorteilhaft ist, wenn nach dem Einbringen der Öffnung in die Zellmembran durch die Öffnung hindurch eine intrazelluläre Manipulation durchgeführt wird, insbesondere ein Medikament und/oder Fremdstoff und/oder eine biologische Substanz in das Innere der Zelle gebracht wird. Die Wirkungen der intrazellulären Manipulation können dann durch Messen einer Zustandgröße der Zelle beobachtet werden. Gegebenenfalls kann die intrazelluläre Manipulation aber auch durchgeführt werden, ohne daß eine Zustandgröße der Zelle durch die Öffnung hindurch gemessen wird.

Bezüglich der Vorrichtung besteht die Lösung der vorstehend genannten Aufgabe darin, daß innerhalb des Auflagebereichs die Meßsonde und ein diese umgrenzender elektrischer Isolator angeordnet sind, derart, daß die Zelle an dem Isolator gegen das Nährmedium dichtend anlagerbar ist, und daß zum Öffnen der Zellmembran der Zelle im Bereich der Meßsonde zumindest ein Porationswerkzeug in dem Auflagebereich angeordnet ist.

Da das Porationswerkzeug innerhalb des Aufnahmebereichs angeordnet ist, kann sich die Zelle selbständig an dem in dem Aufnahmebereich befindlichen Porationswerkzeug anlagern. In vorteilhafter Weise kann dadurch ein aufwendiges manuelles Positionieren des Porationswerkzeuges an der Zelle entfallen. Auch werden keine Hilfsvorrichtungen, wie beispielsweise Mikromanipulatoren benötigt. Dadurch ist es möglich, an mehreren dicht benachbart zu einander in dem Aufnahmebereich angeordneten Zellen gleichzeitig Zellgrößen zu messen. Nach dem Einbringen der Öffnung in die Zellmembran verbleibt der die Öffnung umgebene Rand der Zellmembran mit dem elektrischen Isolator in Berührung und dichtet die Öffnung gegen das Nährmedium ab. Dadurch ist die im Inneren der Zelle befindliche Zellflüssigkeit elektrisch gut gegen das Nährmedium isoliert, so daß die Zellgröße durch die Öffnung der Zellmembran hindurch gemessen werden kann.

Vorteilhaft ist, wenn das Porationswerkzeug im wesentlichen konzentrisch um die Meßsonde herum angeordnet ist. Die Meßsonde kann dann nach dem Einbringen der Öffnung in die Zellmembran besonders gut mit der im Inneren der Zelle befindlichen Zellflüssigkeit in Verbindung gelangen.

Bei einer Ausführungsform, die zum Messen des Zellpotentials der Zelle vorgesehen ist, ist die Meßsonde eine Meßelektrode, der wenigstens eine mit dem Nährmedium in Kontakt bringbare Referenzelektrode zugeordnet ist. Mit einer solchen Vorrichtung kann der Potentialunterschied zwischen der Zellflüssigkeit und dem Nährmedium auf einfache Weise gemessen werden.

Besonders vorteilhaft ist, wenn die Meßelektrode auch eine Elektroporations-Elektrode ist, die zum Elektroporieren der Zellmembran der Zelle mit einer elektrischen Spannungsquelle verbindbar ist, und daß die Elektroporations-Elektrode wenigstens einen innerhalb des Auflagebereichs angeordneten aktiven Elektrodenbereich aufweist, der von dem elektrischen Isolator umgrenzt ist. Die Meßelektrode ist also gleichzeitig auch das Porationswerkzeug, und erfüllt somit eine Doppelfunktion, wodurch sich eine besonders einfach aufgebaute Vorrichtung ergibt. Die an den Meßverstärker angeschlossene Elektrode kann dazu beispielsweise mittels eines elektronischen Schalters kurzzeitig auf das Potential der Spannungsquelle gelegt werden. Gegebenenfalls kann auch ein Umschalter vorgesehen sein, mit dem die Meßelektrode wahlweise nacheinander mit dem Meßverstärker oder der Spannungsquelle verbindbar ist. Die Meß- und Elektroporations-Elektrode ist innerhalb des Auflagebereichs des Objektträgers angeordnet, so daß sich eine an dem Isolator adhärent angelagerte Zelle gegebenenfals auch an dem aktiven Elektrodenbereich der Elektrode anlagern kann oder sich zumindest bis in den Wirkungsbereich eines von der Elektrode ausgehenden elektrischen Feldes an diese annähern kann. Beim Anlegen der Elektroporations-Spannung an die Elektrode fließt ein elektrischer Strom, der in die Zellmembran eine Öffnung einbringt. Dabei verbleibt der die Öffnung umgebene Rand der Zellmembran mit dem elektrischen Isolator in Berührung und dichtet die Öffnung gegen das Nährmedium ab. Der Isolationswiderstand ist abhängig vom Zelltyp und ist vorzugsweise größer als 10 Megaohm. Dadurch wird ein Potentialausgleich zwischen dem Nährmedium und der Zellflüssigkeit weitestgehend unterbunden. Nach dem Einbringen der Öffnung in die Zellmembran wird die Meßelektrode von der Elektroporations-Spannungsquelle getrennt, so daß dann das Zellpotential an der mit der Zellflüssigkeit in Kontakt stehenden Meßelektrode anliegt und mittels des Meßverstärkers gemessen werden kann. Die Vorrichtung weist einen einfachen Aufbau auf und ermöglicht eine weitgehend automatisierte Zellpotentialmessung.

Bei einer vorteilhaften Ausführungsform der Erfindung ist vorgesehen, daß das Porationswerkzeug eine von der Meßsonde beabstandete Elektroporations-Elektrode ist, die zum Elektroporieren der Zellmembran der Zelle mit einer elektrischen Spannungsquelle verbindbar ist, daß die Elektroporations-Elektrode wenigstens einen innerhalb des Auflagebereichs angeordneten, von dem elektrischen Isolator umgrenzten aktiven Elektrodenbereich aufweist. Die Elektroporations-Elektrode ist also von der Meßsonde getrennt. Dadurch wird der Einfluß der Kapazität der Zuleitungen von der Spannungsquelle zu der Elektroporations-Elektrode auf das mit der Meßsonde ermittelte Meßsignal reduziert. Somit können schnelle zeitliche Veränderungen des Zellpotentials, wie sie beispielsweise bei Nervenzellen auftreten, noch besser gemessen werden.

Bei einer besonders vorteilhaften Weiterbildung der Erfindung ist vorgesehen, daß der aktive Elektrodenbereich der Elektroporations-Elektrode mindestens eine scharfe Spitze oder Kante aufweist und vorzugsweise gegenüber der Oberflächenebene des Auflagebereichs vorstehend angeordnet ist. Der aktive Elektrodenbereich kann somit die Zellmembran einer an dem Auflagebereich angelagerten Zelle außenseitig berühren. Dadurch wird ein guter elektrischer Kontakt zwischen der Elektrode und der Zellmembran ermöglicht. Der vorstehende aktive Elektrodenbereich ermöglicht ferner durch die Öffnung der Zellmembran hindurch einen guten elektrischen Kontakt zu der im Inneren der Zelle befindlichen Zellflüssigkeit und wirkt darüber hinaus einem Schließen der durch Elektroporose in die Zellmembran eingebrachten Öffnung durch Zellreparaturmechanismen entgegen.

Vorteilhaft ist, wenn die Elektroporations-Elektrode als Hohlelektrode ausgebildet ist, die eine Innenhöhlung mit einer an der Oberfläche des Auflagebereichs befindlichen Öffnung aufweist, und wenn die Meßelektrode eine in der Innenhöhlung der Elektroporations-Elektrode angeordnete Stabelektrode ist, deren freies Ende vorzugsweise bis an die Öffnung der Innenhöhlung heranreicht. Im Inneren der Hohlelektrode kann ein Elektrolyt angeordnet sein, der beispielsweise dem Nährmedium entsprechen kann, so daß nach dem Öffnen der Zellwand in der Zellflüssigkeit enthaltene Ladungsträger, insbesondere Ionen, durch diesen Elektrolyt hindurch zu der Elektrode gelangen können. Somit kann eine größere Elektrodenoberfläche mit den Ladungsträgern in Berührung geraten.

Der elektrische Kontakt zwischen der Meßelektrode und der Zellflüssigkeit wird dadurch verbessert.

Besonders vorteilhaft ist, wenn unmittelbar benachbart zu der Elektroporations-Elektrode ein Schaltelement, insbesondere ein Halbleiterschalter, angeordnet ist, mit dem die Elektroporations-Elektrode mit der Elektroporations-Spannungsquelle verbindbar ist. Die Verbindungsleitung zwischen Elektroporations-Elektrode und der Elektroporations-Spannungsquelle kann dann dicht benachbart zu der Elektrode unterbrochen werden, so daß die parasitären Kapazitäten dieser Verbindungsleitung während der Messung des Zellpotentials von der Meßsonde entkoppelt sind. Dadurch können zeitlich schnell veränderliche Zellpotentialspannungen genauer gemessen werden. Vorzugsweise ist der Halbleiterschalter ein rauscharmer Junction-Feldeffekt-Transistor.

Bei einer vorteilhaften Ausführungsform der Vorrichtung ist das Porationswerkzeug zum Öffnen der Zellmembran der Zelle mittels wenigstens eines Aktuators, insbesondere eines Piezoelements quer zur Oberfläche des Auflagebereichs relativ zu dem Objektträger bewegbar. Bei dieser Vorrichtung wird die Öffnung also mechanisch in die Zellmembran eingebracht. Dabei kann das Porationswerkzeug sogar wechselweise auf die Zellmembran zu und von dieser wegbewegt werden. Zu diesem Zweck kann der Aktuator mit einer Ansteuereinrichtung zum Erzeugen einer Ultraschallschwingung verbunden sein. Das Porationswerkzeug kann in einer rechtwinklig oder in einer schräg zur Oberfläche des Auflagebereichs verlaufenden Richtung relativ zu dem Objektträger bewegbar sein.

Vorteilhaft ist, wenn das Porationswerkzeug mindestens eine, mit der Zellmembran der Zelle in Berührung bringbare scharfe Spitze oder Kante aufweist. Die Zellmembran kann dann durch Bewegen des Porationswerkzeugs besser geöffnet werden.

Besonders vorteilhaft ist, wenn die Meßsonde gleichzeitig auch das Porationswerkzeug ist und dazu mittels des Aktuators oder des Piezoelements quer zur Oberfläche des Auflagebereichs relativ zu dem Objektträger bewegbar ist. Die Meßsonde erfüllt dann eine Doppelfunktion, wodurch ein zusätzliches Porationswerkzeug eingespart werden kann.

Bei einer anderen vorteilhaften Ausführungsform weist der Objektträger im Bereich der Meßsonde ein optisches Fenster auf, das zum Öffnen der Zellmembran im Strahlengang eines Laserstrahls angeordnet ist. Mit dieser Vorrichtung kann ein Teilbereich der Zellmembran kurzzeitig mit energiereicher optischer Strahlung bestrahlt werden, wobei sich dieser so stark erwärmt, daß eine Öffnung in die Zellmembran eingebracht wird.

Besonders vorteilhaft ist, wenn zum Erzeugen des Laserstrahls eine Laserdiode in den Objektträger integriert ist. Dabei kann die Laserdiode sogar direkt hinter der Öffnung angeordnet sein, so daß die Laserstrahlung unmittelbar und somit weitgehend verlustfrei in die Zellmembran der an dem Auflagebereich angelagerten Zelle eingekoppelt werden kann.

Zweckmäßigerweise ist die Meßsonde im wesentlichen konzentrisch um das optische Fenster herum angeordnet. Die Meßsonde kann dann nach dem Öffnen der Zellmembran besser mit der Zellflüssigkeit in Kontakt geraten.

Bei einer vorteilhaften Ausführungsform ist vorgesehen, daß das Porationswerkzeug zum Öffnen der Zellmembran der Zelle eine chemische Substanz und/oder wenigstens eine mit einem Zuführkanal verbundene Austrittsöffnung für eine chemische Substanz aufweist. Die Öffnung kann also auch chemisch in die Zellmembran eingebracht werden, wobei als Porationsmittel zum Beispiel Perforine oder Triton® verwendet werden können.

Bei einer anderen Ausführungsform weist das Porationswerkzeug wenigstens einen in den Auflagebereich mündenden Kanal auf, mittels dem ein Teilbereich der Zellmembran der Zelle zum Einbringen der Öffnung in die Zellmembran mit einem Unter- und/oder Überdruck beaufschlagbar ist. Bei einer solchen Vorrichtung wird also die Öffnung mittels eines Unter- oder Überdrucks in die Zellmembran eingebracht, wobei der Unter- bzw. Überdruck nach dem Öffnen der Zellmembran abgeschaltet wird. Dadurch wird bei einem Öffnen der Zellmembran durch Unterdruck ein Absaugen von Zellflüssigkeit aus dem Inneren der Zelle weitestgehend vermieden. Enstprechend wird beim Öffnen der Zellmembran mittels Überdruck vermieden, daß ein in dem Kanal befindliches Medium, das vorzugsweise ein Fluid ist, in das Innere der Zelle gelangen kann. Zum Abschalten des Unter- bzw. Überdrucks kann beispielseise eine beim Öffnen der Zellmembran in dem Kanal auftretende Druckveränderung ermittelt werden. Der Unter- bzw. Überdruck kann mit einer geeigneten Hifsvorrichtung, beispielsweise einer Pumpe, oder hydrostatisch, erzeugt werden. In vorteilhafter Weise kann der Kanal vor dem Anlagern der Zelle auch dazu genutzt werden, um Nährmedium aus dem Auflagebereich abzusaugen, so daß in dem Nährmedium eine Strömung entsteht, welche die darin befindlichen Zellen zu der im Bereich der Meßelektrode angeordneten Mündung des Kanals leitet.

Besonders vorteilhaft ist, wenn die Meßsonde als in die Oberfläche des Objektträgers eingelassene, wenigstens eine Innenhöhlung aufweisende Hohlsonde ausgebildet ist, und daß die Innenhöhlung an der Oberfläche des Auflagebereichs eine Öffnung aufweist. Im Inneren der Hohlelektrode kann ein Elektrolyt angeordnet sein, so daß in der Zellflüssigkeit enthaltene Ladungsträger nach dem Öffnen der Zellmembran durch den Elektrolyt hindurch zu der Meßsonde gelangen können. Somit kann eine größere Oberfläche der Meßsonde mit den Ladungsträgern in Berührung geraten, was den elektrischen Kontakt zwischen der Meßelektrode und der Zellflüssigkeit verbessert.

Eine vorteilhafte Ausführungsform sieht vor, daß der elektrische Isolator innerhalb des Auflagebereichs einen gegenüber dessen Oberflächenebene vorstehenden Vorsprung aufweist, und daß die Meßsonde an dem der Oberfläche des Auflagebereichs abgewandten freien Ende des Vorsprungs angeordnet ist. Dadurch ergibt sich ein guter elektrischer und/oder mechanischer Kontakt zwischen der Meßsonde und der Zellmembran.

Zweckmäßigerweise ist vorgesehen, daß sich der Querschnitt des Vorsprungs ausgehend von der Oberflächenebene des Auflagebereichs zu der am weitesten vorstehenden Stelle verjüngt. Die Zelle haftet dann mit ihrer Membran besser an dem Vorsprung des elektrischen Isolators an. Außerdem kann der Isolator bei der Herstellung des Objektträgers als Beschichtung fertigungstechnisch besser auf dem sich verjüngenden Bereich des Vorsprungs aufgetragen werden.

Bei einer vorteilhaften Weiterbildung der Erfindung ist vorgesehen, daß der Objektträger im Auflagebereich eine Profilierung aufweist, die wenigstens eine um die Meßsonde umlaufende Profilierungsvertiefung und/oder einen um die Meßsonde umlaufenden Profilierungsvorsprung hat. Dadurch wird eine bessere Abdichtung der Zellflüssigkeit gegen das Nährmedium durch die an dem Isolator anhaftende Zellmembran erreicht.

Vorteilhaft ist, wenn die Profilierungsvertiefung und/oder der Profilierungsvorsprung in Erstreckungsrichtung durch wenigstens eine Unterbrechung unterbrochen ist. Die Zelle kann dann im Bereich der Profilierung besser an der Oberfläche des Objektträgers anhaften. Der Profilierungsvorsprung bzw. die Profilierungsvertiefung können beispielsweise eine Wabenstruktur oder eine Struktur nach Art eines Karo- oder Schachbrettmusters aufweisen.

Besonders vorteilhaft ist, wenn die Profilierungsvertiefung und/oder der Profilierungsvorsprung ringförmig ausgebildet ist und wenn vorzugsweise mehrere solcher ringförmiger Profilierungsvertiefungen und/oder Profilierungsvorsprünge im wesentlichen konzentrisch zur Meßsonde angeordnet sind. Somit sind radial zur Meßsonde mehrere Profilierungsvertiefungen und/oder -vorsprünge hintereinander geschaltet bzw. ineinander verschachtelt, so daß die Zellflüssigkeit noch besser gegen das Nährmedium abgedichtet ist.

Eine bevorzugte Ausführungsform der Erfindung sieht vor, daß der Isolator eine an der Oberfläche der Profilierung angeordnete Isolationsschicht ist. In vorteilhafter Weise wird durch die so profilierte Isolationsschicht der Weg für einen an der Oberfläche des Isolators von der Zellflüssigkeit zu dem Nährmedium fließenden Kriechstrom vergrößert, so daß die Meßsonde bzw. die Meßelektrode noch besser gegen das Nährmedium isoliert ist.

Eine andere Ausführungsform sieht vor, daß der (die) Profilierungsvorsprung (-vorsprünge) auf die Oberfläche des Isolators aufgebracht ist (sind). Der Objektträger ist dann fertigungstechnisch einfacher herstellbar.

Besonders vorteilhaft ist, wenn im Auflagebereich des Objektträgers an dessen Oberfläche eine wenigstens ein Zelladhäsionsprotein aufweisende Beschichtung und/oder eine hydrophile Beschichtung angeordnet ist. Die Zellmembran der Zelle haftet dann besser an dem Objektträger an. Die Zelladhäsions-Beschichtung kann beispielsweise Laminin, Fibronectin oder Poly-L-Lysin aufweisen. Gegebenenfalls kann an dem an die Elektrode angrenzenden Rand des Auflagebereichs auch eine hydrophobe Beschichtung mit Bindungsstellen für in der Zellmembran befindliche hydrophobe Lipide angeordnet sein.

Vorteilhaft ist, wenn als mechanische Führung für die Zellen beidseits der Meßsonde Begrenzungswände angeordnet sind, die vorzugsweise einen nutenartigen Führungskanal begrenzen. Dabei ist (sind) die Meßelektrode(n) vorzugsweise mittig zwischen den Begrenzungswänden am Nutgrund des Führungskanal angeordnet, so daß in dem Führungskanal befindliche Zellen sich im wesentlichen nur in Erstreckungsrichtung des Führungskanals bewegen können und dann zwangsläufig mit der Meßsonde in Berührung geraten.

Bei einer besonders vorteilhaften Weiterbildung der Erfindung ist benachbart zu der Meßsonde ein Feldeffekt-Transistor (FET), insbesondere ein Junction-Feldeffekt-Transistor (J-FET) angeordnet und die Meßsonde ist zur Impedanzwandlung des Meßsignales mit dem Gate des FET verbunden. Dabei erfolgt die Ankopplung der Meßelektrode an das Gate bei einem Metalloxid-Feldeffekt-Transistor (MOS-FET) kapazitiv, wobei die Meßelektrode vorzugsweise unmittelbar über dem Gate des in die Oberfläche des Objektträgers eingelassenen MOS-FET angeordnet ist. In vorteilhafter Weise ermöglicht ein Junction-FET eine hochohmige, aber dennoch rauscharme Auskopplung eines intrazellulären elektrischen Signales. Die niedrige Eingangskapazität des Junction-FET ermöglicht insbesondere auch bei schnellen Zellpotentialveränderungen eine weitgehend rückwirkungsfreie Meßsignalgewinnung. Durch die Impedanzwandlung direkt am Meßort kann der Abschirmungsaufwand für die Verbindungsleitungen von der Meßelektrode zu einem Meßverstärker und/oder einer Auswerteeinrichtung reduziert werden. Außerdem wird die Beeinflussung des Meßsignales durch parasitäre Kapazitäten in der Verbindungleitung vermindert. Die mit bekannten halbleitertechnologischen Fertigungsverfahren herstellbaren Feldeffekt-Transistoren ermöglichen darüber hinaus eine hohe Integrationsdichte.

Vorteilhaft ist, wenn in dem Auflagebereich des Objektträgers benachbart zu der Meßsonde, vorzugsweise in einem von dieser umgrenzten Bereich wenigstens ein Fluidkanal mündet. Durch diesen Fluidkanal kann dann nach dem Öffnen der Zellmembran eine kleine Menge Zellflüssigkeit abgesaugt und/oder eine biologische Substanz, beispielsweise ein Gen, und/oder ein Fremdstoff, ein Medikament oder dergleichen, dessen Wirkung auf die Zelle untersucht werden soll, der Zellflüssigkeit zugegeben werden. Zur Zugabe eines Stoffes kann die Vorrichtung ggf. auch ohne eine Meßsonde verwendet werden.

Zweckmäßigerweise ist im Verlauf des Fluidkanals eine vorzugsweise in den Objektträger integrierte Mikropumpe angeordnet. Gegebenenfalls können einem Fluidkanal auch mehrere Mikropumpen zugeordnet sein, die beispielsweise jeweils mit einer in dem Objektträger befindlichen Kavität zum Deponieren einer zytologisch zu untersuchenden Flüssigkeit oder eines Mediums verbunden sein können.

Besonders vorteilhaft ist, wenn innerhalb des Fluidkanals, vorzugsweise in einer Wandung des Fluidkanals, wenigstens ein Mikrosensor zum Messen einer Zellgröße der Zelle angeordnet ist. Dadurch können zusätzliche intrazelluläre Parameter, wie beispielsweise Ionenkonzentrationen, Gasgehalte, Enzym- und/oder Proteinkonzentrationen, ermittelt werden.

Vorteilhaft ist, wenn zum Erzeugen eines die Zelle zu der Meßsonde leitenden elektrischen Feldes im Auflagebereich und/oder benachbart dazu wenigstens eine Zusatzelektrode angeordnet ist. Dadurch kann an der Oberfläche des Objektträgers ein elektrisches Feld erzeugt werden, das auf biologische Zellen, deren Dielektrizitätskonstante sich von derjenigen des Nährmediums, in dem sie angeordnet sind, unterscheidet, eine Kraft ausübt, welche die Zellen zu der Meßsonde leitet.

Eine besonders vorteilhafte Weiterbildung der Erfindung sieht vor, daß in dem Auflagebereich mehrere Meßsonden und Elektroporations-Elektroden vorzugsweise als Array angeordnet sind und daß diesen Meßsonden jeweils wenigstens ein Porationswerkzeug zugeordnet ist. Eine solche Vorrichtung ermöglicht eine ortsaufgelöste Messung des Zellpotentials an einer Zellpopulation. Dabei kann an einer Vielzahl dicht zueinander benachbarter Zellen gleichzeitig das Zellpotential gemessen werden. Dadurch ist es beispielsweise möglich, an Nerven- oder Tumorzellen quasi statische Zellmembran-Potentialmessungen durchzuführen, um deren elektrische Aktivität zu überwachen. Dabei ist es sogar möglich, in einem Zellverbund mit durch Synapsen verbunden Nervenzellen, die Informationsübermittlung zwischen den Zellen durch orts- und zeitaufgelöstes Messen der Zellpotentiale zu überwachen. Gegebenenfalls kann in den Objektträger auch ein Multiplexer integriert sein, mit dem eine Vielzahl von Meß- und/oder Elektroporations-Elektroden wechselweise nacheinander mit einer Meßsignalleitung verbindbar sind, wodurch sich die Anzahl der Zuleitungen zu dem als Sensorchip ausgebildeten Objektträger entsprechend reduziert.

Nachfolgend sind Ausführungsbeispiele der Erfindung anhand der Zeichnung näher erläutert. Es zeigen:
- Fig.1: einen Längsschnitt durch eine im Auflagebereich eines Objektträgers angeordnete Meß- und Elektroporationselektrode, die mittels eines Umschalters wahlweise mit einer Spannungsquelle und einem Meßverstärker verbindbar ist,
- Fig.2: eine Aufsicht auf die Meßelektrode gem. Fig.1,
- Fig.3: eine Darstellung ähnlich Fig.1, wobei jedoch für die Messung und die Elektroporation getrennte Elektroden vorgesehen sind,
- Fig.4: eine Aufsicht auf die Meß- und Elektroporationselektrode gem. Fig.3,
- Fig.5: eine Darstellung ähnlich Fig.1, wobei jedoch die Elektrode als kegelstumpfförmige Hohlelektrode mit zylindrischer Innenhöhlung ausgebildet ist,
- Fig.6: eine Aufsicht auf die Elektrode gem. Fig.5,
- Fig.7: eine Darstellung ähnlich Fig.5, wobei jedoch die Elektrode eine im wesentlichen zylindrische Form aufweist,
- Fig.8: eine Aufsicht auf die Elektrode gem. Fig.7,
- Fig.9: eine Vorrichtung, bei der die Meß- und Elektroporations-Elektrode elektrisch leitend an das Gate eines J-FET angekoppelt ist,
- Fig.10: eine Darstellung ähnlich Fig.9, wobei jedoch benachbart zu der Elektrode ein als Schalter dienender, weiterer Feldeffekt-Transistor angeordnet ist, über den die Elektrode mit einer Elektroporations-Spannungsquelle verbindbar ist,
- Fig.11: eine Vorrichtung mit einem eine Elektrode aufweisenden Objektträger, auf dem eine in einem Nährmedium befindliche biologische Zelle adhärent angelagert ist,
- Fig.12: eine Aufsicht auf den in Fig.11 gezeigten Objektträger,
- Fig.13: einen Längsschnitt durch einen Objektträger, der um die Elektrode herum eine Oberflächenstrukturierung aufweist, auf die eine elektrische Isolationsschicht aufgebracht ist,
- Fig.14: eine Aufsicht auf den Objektträger gem. Fig.13,
- Fig.15 und 16: eine Ansicht ähnlich Fig. 14, wobei jedoch der Objektträger eine andere Oberflächenprofilierung aufweist,
- Fig.17: eine Aufsicht auf einen Objektträger, der ein Array mit mehreren Meß- und Elektroporations-Elektroden aufweist,
- Fig.18: einen Längsschnitt durch einen Objektträger, der eine zwischen zwei Begrenzungswänden innerhalb einer Oberflächenstrukturierung angeordnete Elektrode aufweist,
- Fig.19: einen Längsschnitt durch eine im Auflagebereich eines Objektträgers angeordnete, mittels eines Piezoelements bewegbare Meß- und Elektroporationselektrode,
- Fig.20: einen Längsschnitt durch eine im Auflagebereich eines Objektträgers angeordnete, ein optisches Fenster aufweisende Elektroporationselektrode, hinter dem eine Laserdiode angeordnet ist,
- Fig.21: eine Darstellung ähnlich Fig.20, wobei jedoch ein externer Laser verwendet wird, dessen Laserstrahl in das optische Fenster eingekoppelt wird und
- Fig.22: eine Darstellung ähnlich Fig.5, wobei jedoch durch die Elektrode hindurch ein Fluidkanal zu dem Auflagebereich führt und wobei im Verlauf des Fluidkanals mehrere Meßsonden angeordnet sind.

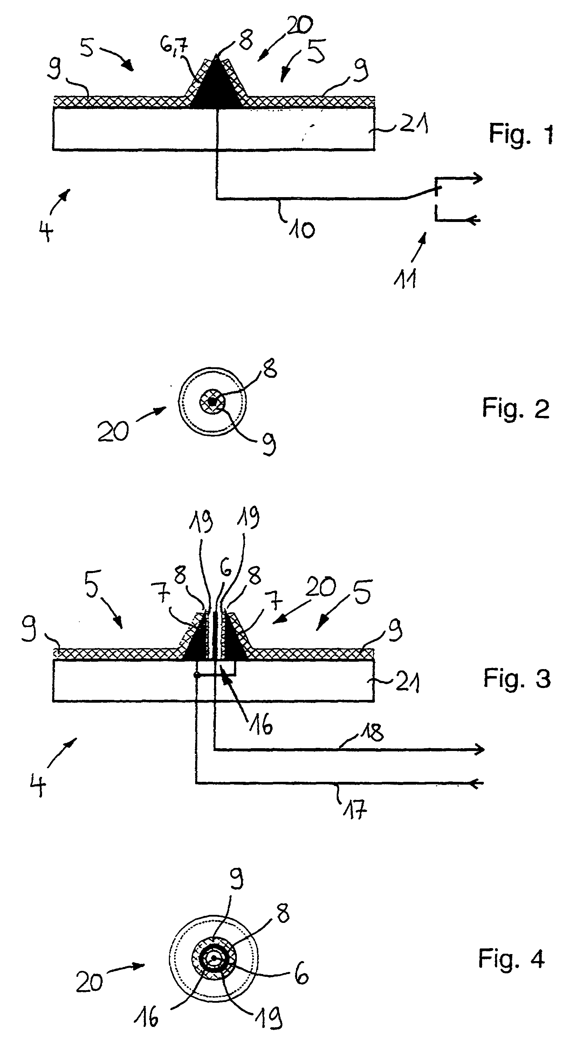

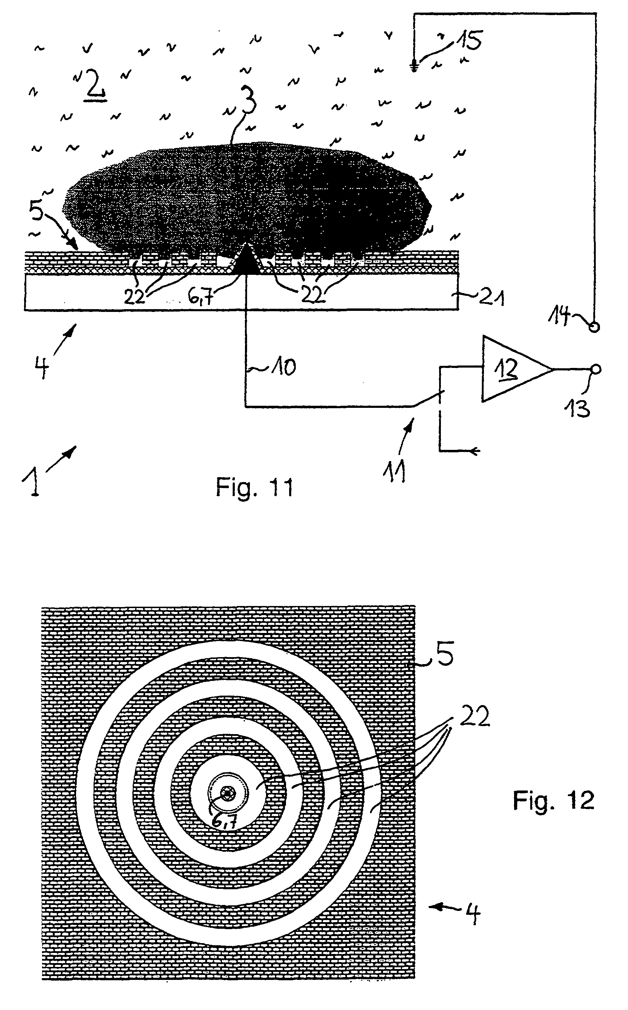

Eine im ganzen mit 1 bezeichnete Vorrichtung zur Messung des Zellpotentials einer in einem Nährmedium 2 befindlichen biologischen Zelle 3 (Fig.11) weist einen Objektträger 4 auf, der einen Auflagebereich 5 hat, an den die Zelle 3 adhärent anlagerbar ist. Die Zelle 3 ist also auf dem Objektträger 4 immobilisiert und haftet an dem Auflagebereich 5 an. Bei den Ausführungsbeispielen nach Fig.1 und 2 sowie 5 bis 17 weist der Objektträger 4 innerhalb des Auflagebereichs 5 eine Meß- und Elektroporations-Elektrode 6/7 auf, die einen gegenüber der Oberflächenebene des Auflagebereichs 5 vorstehenden aktiven Elektrodenbereich 8 hat. In dem Auflagebereich 5 ist ein den aktiven Elektrodenbereich 8 umgrenzender elektrischer Isolator 9 angeordnet, an dem die Zelle 3 gegen das Nährmedium 2 abdichtend anlagerbar ist.

Bei den Ausführungsbeispielen gemäß Fig.1, 5, 7 und 11 ist die Elektrode mittels einer in den Objektträger integrierten Leiterbahn 10 an einem Umschalter 11 angeschlossen, mit dem sie wahlweise mit einem Meßverstärker und einer Elektroporations-Spannungsquelle verbindbar ist. Zum Messen des Zellpotentiales wird die Elektrode 6/7 zunächst mit der im Inneren der Zelle 3 befindlichen Zellflüssigkeit in Berührung gebracht. Dazu wird zwischen der Elektrode 6/7 und dem Nährmedium 2 eine elektrische Spannung angelegt, indem die Elektrode 6/7 über den Umschalter 11 mit der Elektroporations-Spannungsquelle verbunden wird. Dabei fließt über die Elektrode 6/7 ein elektrischer Strom in die Zellmembran, wodurch im Bereich der Elektrode 6/7 eine Öffnung in die Zellmembran eingebracht wird und der aktive Elektrodenbereich 8 durch diese Öffnung hindurch in die Zelle 3 eindringt. Dabei gerät die Elektrode 6/7 mit der Zellflüssigkeit in Berührung. Nach dem Einbringen der Öffnung in die Zellmembran wird die Elektrode 6/7 mit dem Eingang des Meßverstärkers 12 verbunden. Der Ausgang des Meßverstärkers 12 ist mit einem Anschlußkontakt 13 verbunden. Ein weiterer Anschlußkontakt 14 ist mit einer Referenzelektrode 15 verbunden, die elektrisch leitend mit dem Nährmedium 2 in Kontakt steht. Zwischen den Anschlußkontakten 13 und 14 liegt eine elektrische Spannung an, die ein Maß für das Zellpotential der Zelle 3 ist. An den Anschlußkontakten 13 und 14 kann zum Beispiel eine Anzeigeund/oder Auswerteeinrichtung angeschlossen werden. Die in die Zelle 3 mittels der Elektroporations-Elektrode 6/7 eingebrachte Öffnung ist durch den sie umschließenden an dem Objektträger 4 anhaftenden Zellmembranbereich gegen das Nährmedium 2 abgedichtet. Dadurch wird ein Potentialausgleich zwischen dem Potential der Elektrode 6/7 und dem des Nährmediums 2 verhindert.

Bei den Ausführungsbeispielen nach Fig.1, 9 bis 11 und 13 ist die Elektrode 6/7 etwa kegelförmig ausgebildet, wobei der gegenüber der Oberflächenebene des Auflagebereichs vorstehende, aktive Elektrodenbereich 8 an der Spitze des Kegels angeordnet und als scharfe Spitze ausgebildet ist. Dadurch entsteht beim Anlegen einer Elektroporations-Spannung an die Elektrode 6/7 in dem aktiven Elektrodenbereich 8 eine besonders hohe elektrische Feldstärke.

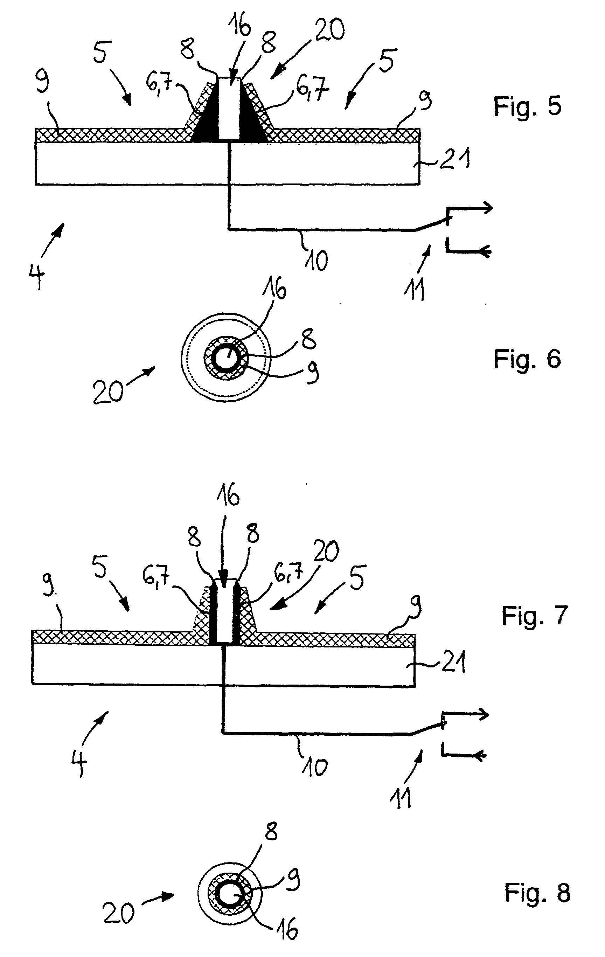

Bei den Ausführungsbeispielen gemäß Fig.5 bis 8 ist die Elektrode 6/7 eine Hohlelektrode, die mit ihrem aktiven Elektrodenbereich 8 über die Oberfläche des Auflagebereichs 5 vorstehend in die Oberfläche des Objektträgers 4 eingelassen ist. Dabei ist die Elektrode 6/7 gemäß Fig.5 im wesentlichen kegelstumpfförmig und die Elektrode 6/7 gemäß Fig. 7 im wesentlichen als zylindrische Hülse ausgebildet, wobei die Symmetrieachse der Elektrode 6/7 jeweils etwa rechtwinklig zur Oberflächenebene des Objektträgers 4 im Auflagebereich 5 angeordnet ist. Die Hohlelektrode weist eine mit dem Nährmedium 2 gefüllte Innenhöhlung 16 auf, die an der Oberfläche des Auflagebereichs 5 eine Öffnung hat. Durch diese Öffnung hindurch können bei der an der Zellmembran-Öffnung angeordneten Elektrode 6/7 elektrische Ladungsträger aus der Zellflüssigkeit in die Innenhöhlung 16 gelangen und mit der an die Innenhöhlung 16 angrenzenden Innenwand der Elektrode 6/7 in Berührung geraten. Gegenüber den Ausführungsbeispiel nach Fig. 1 ergibt sich dadurch eine größere aktive Meßelektrodenoberfläche, wodurch sich der elektrische Kontaktwiderstand zwischen der Elektrode 6/7 und der Zellflüssigkeit verringert.

Wie aus Fig.5 und 7 besonders gut erkennbar ist, weist das die Öffnung umgrenzende freie Ende der Elektrode 6/7 eine scharfe Ringkante auf, deren Querschnitt als Spitze ausgebildet ist und sich ausgehend von der Oberflächenebene des Objektträgers zu der am weitesten vorstehenden Stelle der Elektrode 6/7 verjüngt. Dadurch ergibt sich beim Anlegen einer elektrischen Spannung an der Elektroporations-Elektrode 6/7 in dem aktiven Elektrodenbereich 8 eine vergleichsweise hohe Feldstärke, die das Öffnen der Zellmembran erleichtert.

Bei dem Ausführungsbeispiel nach Fig.3 und 4 sind in dem Auflagebereich 5 des Objektträgers 4 eine Meßelektrode 6 und eine davon getrennte Elektroporations-Elektrode 7 angeordnet. Die Elektroporations-Elektrode 7 ist zum Einbringen einer Öffnung in die Zellmembran einer an dem Auflagebereich 5 anhaftenden Zelle mittels einer in den Objektträger 4 integrierten Leiterbahn 17 mit einer Elektroporations-Spannungsquelle verbunden. Eine weitere Leiterbahn 18 verbindet die Meßelektrode 6 mit dem Eingang eines Meßverstärkers. Im übrigen entspricht der Aufbau weitgehend dem Objektträger gemäß Fig.5. Die Elektroporations-Elektrode 7 hat etwa die Form eines Kegelstumpfs und weist eine zylindrische Innenhöhlung auf, deren Zylinderachse etwa mit der Symmetrieachse des Kegelstumpfs übereinstimmt. An dem freien Ende der Elektroporations-Elektrode 7 weist die Innenhöhlung eine zu der Oberfläche des Objektträgers 4 führende Öffnung auf. Die Meßelektrode 6 ist als Stabelektrode ausgebildet, die etwa konzentrisch in der Innenhöhlung der Elektroporations-Elektrode 7 angeordnet ist und mit ihrem freien Ende bis an die Öffnung der Innenhöhlung heranreicht. Um die Meßelektrode 6 von den parasitären Kapazitäten der Elektroporations-Elektrode 7 und deren Zuleitung zu entkoppeln und den elektrischen Widerstand zwischen der Meßelektrode 6 und der Elektroporations-Elektrode 7 zu vergrößern, kann auf der die Innenhöhlung 16 begrenzenden Innenwand der Elektroporations-Elektrode 7 eine Isolationsschicht 19 angeordnet sein, welche die in der Innenhöhlung 16 befindliche Zellflüssigkeit gegen die Elektroporations-Elektrode 7 elektrisch isoliert.

Erwähnt werden soll noch, daß die Meßelektrode 6 und/oder die die Innenhöhlung 16 begrenzende Wand der Meß- und Elektroporations-Elektrode 6/7 eine Oberflächenrauhigkeit aufweisen kann, welche die Oberfläche der Elektrode vergrößert. Die Elektrode 6/7 kann beispielsweise aus porösem Silizium bestehen oder eine Beschichtung aus diesem Material aufweisen.

Wie in Fig. 1 bis 10 besonders gut erkennbar ist, weist der elektrische Isolator 9 innerhalb des Auflagebereichs 5 einen gegenüber dessen Oberflächenebene vorstehenden Vorsprung 20 auf, an dessen der Oberflächenebene abgewandten freiem Ende der aktive Elektrodenbereich der Elektroporations-Elektrode 7 angeordnet ist. Der aktive Elektrodenbereich 8 ist dadurch elektrisch gut leitend an die an dem Auflagebereich anhaftende Zelle 3 angekoppelt. Der Querschnitt des Vorsprungs 20 verjüngt sich ausgehend von der Oberflächenebene des Auflagebereichs 5 zu der am weitesten vorstehenden Stelle hin. Dadurch sind der Vorsprung 20 und die Elektrode 6/7 fertigungstechnisch besser herstellbar. Außerdem weist der sich verjüngende Vorsprung 20 eine gute mechanische Festigkeit auf.

Der Vorsprung 20 kann aber auch einen in seiner Erstreckungsrichtung konstanten oder ausgehend von der Oberflächenebene des Auflagebereichs 5 zu der am weitesten vorstehenden Stelle hin abnehmenden Querschnitt aufweisen. Ein solcher Vorsprung 20 kann beispielsweise mittels LIGA-Technik hergestellt werden.

Erwähnt werden soll noch, daß der Objektträger 4 ein im wesentlichen plattenförmiges Substrat 21 aufweist, das beispielsweise aus einem Halbleitermaterial (z.B. Silizium oder Gallium-Arsenid), Siliziumcarbid, Glas oder Kunststoff bestehen kann. Auf dieses Substrat kann der Isolator 9 als Beschichtung, beispielsweise durch Sputtern aufgebracht sein. Gegebenenfalls kann das Substrat 21 auch eine flexible Folie sein.

Bei den Ausführungsbeispielen gemäß Fig.11 bis 18 weist der Objektträger 4 im Auflagebereich 5 jeweils mehrere, um die Elektroporations-Elektrode 7 umlaufende Profilierungsvertiefungen 22 auf. Wie aus Fig.11 besonders gut erkennbar ist, wird dadurch die Abdichtung der Zellmembran der Zelle 3 gegen den Auflagebereich 5 des Objektträgers 4 verbessert.

Bei dem Ausführungsbeispiel nach Fig.11 bis 14 sind die Profilierungsvertiefungen geschlossene Ringnuten, die konzentrisch zu der Elektroporations-Elektrode 7 angeordnet sind. Die Ringnuten weisen jeweils einen etwa rechteckförmigen Querschnitt auf. Zueinander benachbarte Ringnuten sind jeweils in etwa gleichen Abständen zueinander angeordnet (Fig.12). Die Abstände zueinander benachbarter Profilierungsvertiefungen 22 und die Tiefe dieser Profilierungsvertiefungen 22 sind an den Typ der an dem Auflagebereich 5 anzulagernden Zellen 3 angepaßt. Die Kanten der Profilierungsvertiefungen 22 können gerundet sein, um das adhärente Anlagern einer Zelle 3 zu erleichtern.

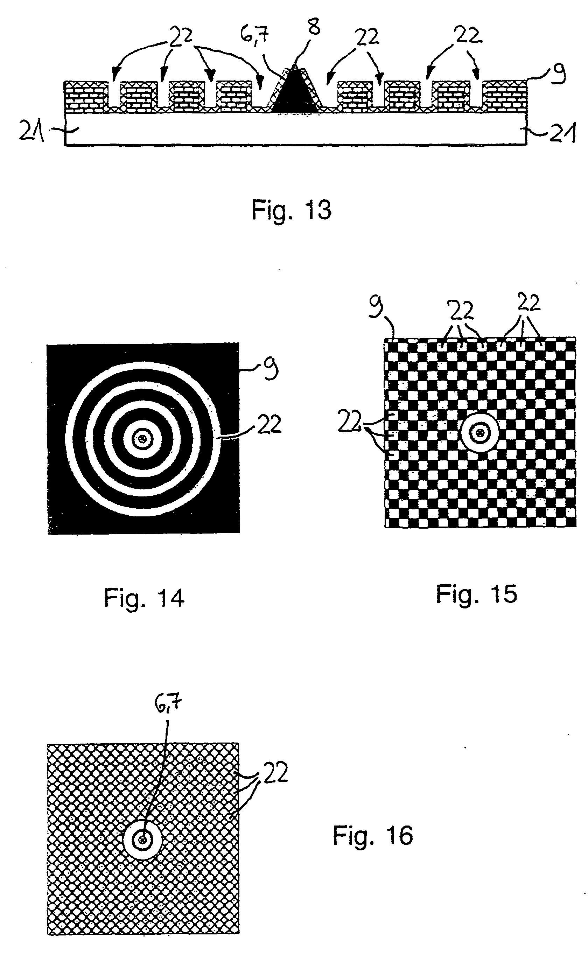

Die Profilierungsvertiefungen 22 können in ihrem Verlauf Unterbrechungen aufweisen, wie dies am Beispiel einer Karo-Strukturierung in Fig.15 und einer Wabenstruktur in Fig.16 gezeigt ist. Die Oberflächenprofilierungen gemäß Fig.14 bis 16, die Oberflächenrauhigkeit und das Oberflächenmaterial können jeweils an einen bestimmten Zelltyp angepaßt sein. Dadurch kann die Zelladhäsion verbessert oder gesteuert werden.

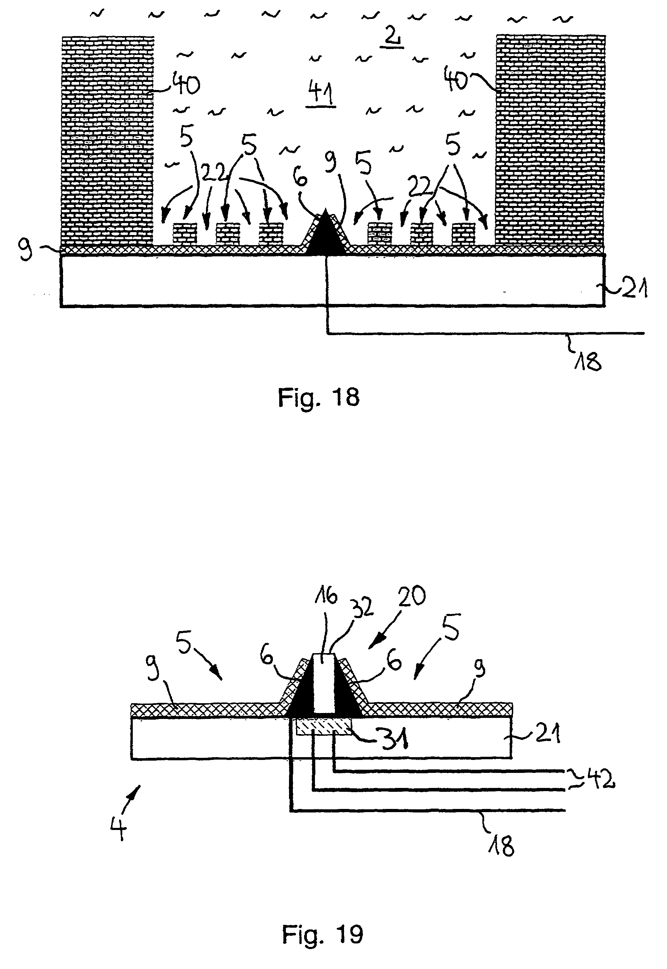

Bei den Ausführungsbeispielen nach Fig.11 und 18 ist die Oberflächen-Profilierung als Beschichtung mit Methoden der Halbleitertechnik auf den elektrischen Isolator 9 aufgebracht. Der Objektträger 4 ist dadurch als Halbleiterchip auf einfache Weise herstellbar. Die Oberflächen-Profilierung kann aber auch mit anderen Verfahren, beispielsweise in Dickschichttechnik, auf den Isolator 9 aufgebracht werden. Bei dem Ausführungsbeispiel gemäß Fig.13 ist die Oberflächenprofilierung eine auf das Substrat 21 aufgebrachte Schicht, die durch eine Isolationsschicht abgedeckt ist, die den Isolator 9 bildet. Durch diese Maßnahme können eventuelle Kriechströme, die von der Elektroporations-Elektrode 7 entlang der Oberfläche des Isolators 9 zu dem Nährmedium 2 fließen, abgeschwächt werden. Dadurch ergibt sich ein entsprechend hoher elektrischer Widerstand zwischen der Elektrode 6/7 und dem Nährmedium 2, wenn an dem Auflagebereich 5 eine Zelle 3 angelagert ist.

Die Herstellung der in Fig.13 gezeigten Oberflächenprofilierung kann beispielsweise in der Weise erfolgen, daß auf das Substrat 21 mittels Maskentechnik eine Ringstruktur aufgebracht wird, die anschließend mit der Isolationsschicht beschichtet wird. Die Profilierung weist dann im Vergleich zu dem Ausführungsbeispiel nach Fig.11 etwas gerundetere Kanten auf. Die Zellen 3 können sich dann besser anlagern.

Bei dem Ausführungsbeispiel gemäß Fig.18 sind beidseits der Elektrode Begrenzungswände 40 angeordnet, die zusammen mit dem Isolator 9 einen im Querschnitt etwa U-förmigen Führungskanal 41 bilden. Dabei sind die Profilierungen 22 und die Meßelektrode am Boden des Führungskanals 41 zwischen den Begrenzungswänden 40 angeordnet. Die Begrenzungswände 40 bilden ein Hindernis für in dem Führungskanal 41 befindliche Zellen 3, das diese nicht oder nicht ohne weiteres überwinden können. Die Zellen 3 können sich dadurch im wesentlichen nur in Erstreckungsrichtung des Führungskanals 41 bewegen, wobei sie zwangsläufig mit der Meßelektrode 6 in Berührung geraten. Der lichte Abstand der beidseits der Meßelektrode 6 angeordneten Begrenzungswände 40 ist an die Abmessungen der Zellen 3 angepaßt und ist vorzugsweise etwas größer gewählt als der Zelldurchmesser der Zellen 3. Gegebenenfalls können mehrere Meßelektroden 6 in Erstreckungsrichtung des Führungskanals 41 hintereinander angeordnet sein. Dadurch kann an mehreren Zellen 3 gleichzeitig das Zellpotential gemessen werden. Der Querschnitt des Führungskanals 41 kann sich in Erstreckungsrichtung verjüngen oder erweitern, d.h. der Führungskanal 41 kann an unterschiedlichen Stellen eine unterschiedliche Breite und/ oder unterschiedliche Querschnittsabmessungen aufweisen. Ausgehend von der tiefsten zu der am weitesten vorstehenden Stelle des Führungskanals 41 kann sich der Querschnitt des Führungskanals 41 beispielsweise verjüngen.

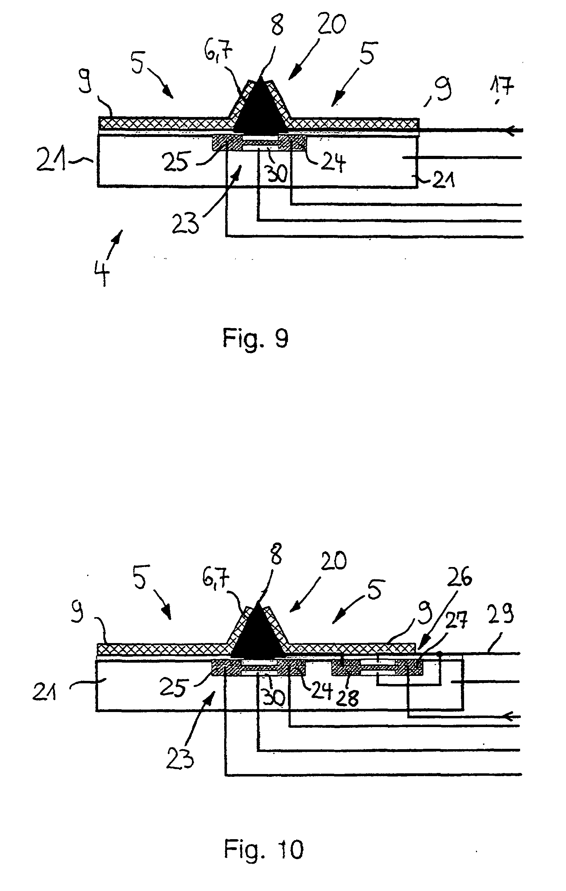

Bei dem Ausführungsbeispiel nach Fig. 9 ist in das Substrat 21 des Objektträgers 4 ein Junction-Feldeffekt-Transistor (J-FET) 23 integriert. Direkt über dem Gate des J-FET ist die Meß- und Elektroporations-Elektrode 6/7 angeordnet, die galvanisch leitend mit dem Gate verbunden ist. Eine zusätzliche Gate-Elektrode 30 ermöglicht eine externe Steuerung der Kanalleitfähigkeit, was eine Arbeitspunkteinstellung ermöglicht. Source 24 und Drain 25 des J-FET 23 sind über in den Objektträger 4 integrierte Leiterbahnen mit einer Auswerteeinrichtung verbunden. Der J-FET 23 weist ein sehr niedriges Rauschen, eine hohe Eingangsimpedanz sowie eine niedrige Eingangskapazität auf und bewirkt eine Impedanzwandlung des aus der Zelle 3 ausgekoppelten elektrischen Signales. Durch den J-FET 23 ist die Meß- und Elektroporations-Elektrode 6/7 von den Leitungskapazitäten der mit dem Source 24 und dem Gate 25 verbundenen Leiterbahnen weitgehend entkoppelt. Dadurch können hochfrequente Signalanteile des Zellpotential-Signales besser gemessen werden.

Zum Elektroporieren der Zellmembran ist die Elektroporations-Elektrode 7 mit einer in den Objektträger 4 integrierten Leiterbahn 17 mit einer Elektroporations-Spannungsquelle verbindbar. Anstelle der Einkopplung der Elektroporationsspannung über die Leiterbahn 17 kann die Elektroporationsspannung auch kapazitiv über das Gate des J-FET 23 in die Elektrode 6/7 eingekoppelt werden, indem die an der Source 24 und dem Drain 25 angeschlossenen Leiterbahnen und gegebenenfalls das Substrat 21 mit der Elektroporations-Spannungsquelle verbunden werden. In diesem Fall kann die Leiterbahn 17 entfallen.

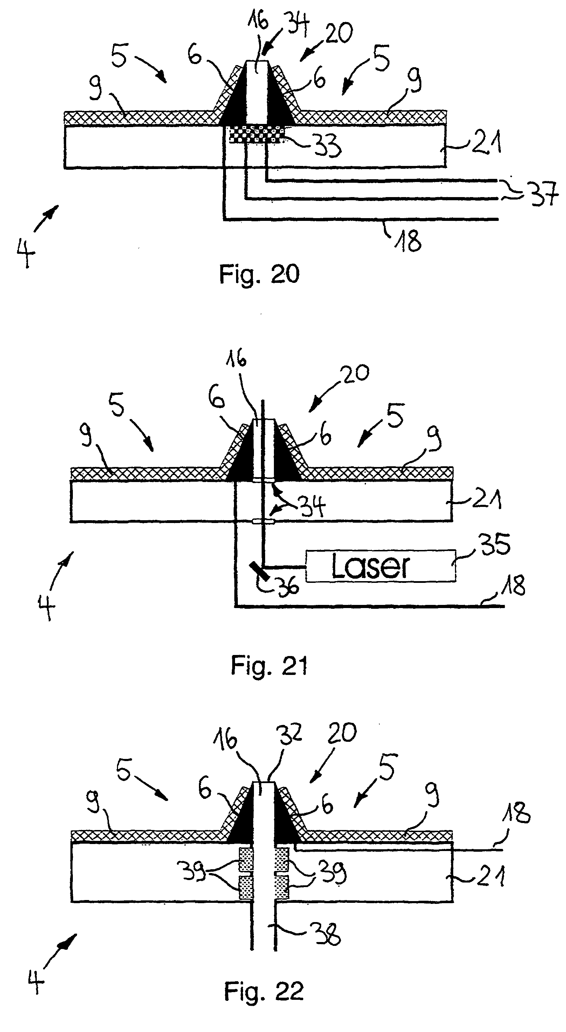

Bei dem Ausführungsbeispiel nach Fig.20 ist dicht benachbart zu dem J-FET 23 ein Schalt-FET 26 in den Objektträger 4 integriert. Der Drain-Anschluß 27 dieses Schalt-FETs 26 ist mit der Elektroporations-Spannungsquelle und der Source-Anschluß 28 mit der Elektrode 6/7 verbunden. Zum Anlegen der Elektroporations-Spannung an die Elektrode 6/7 ist das Gate des Schalt-FET 26 mit einer Steuerleitung 29 verbunden, an die eine Steuerspannung anlegbar ist. In vorteilhafter Weise ist bei gesperrter Source-Drain-Verbindung die Elektrode 6/7 weitestgehend gegen Streukapazitäten und Einkopplungen der Verbindungsleitung zu der Elektroporations-Spannungsquelle entkoppelt.

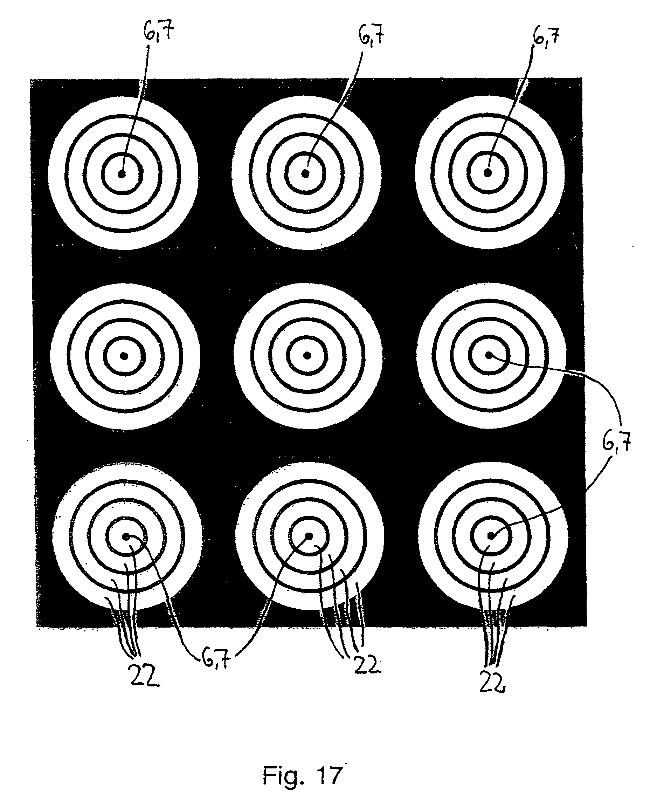

Bei dem Ausführungsbeispiel gemäß Fig.17 sind im Auflagebereich 5 des Objektträgers 4 mehrere Meß- und Elektroporations-Elektroden 6/7 in Form eines Arrays angeordnet. Die einzelnen Elektroden 6/7 sind jeweils an Rasterpunkten eines karthesischen Koordinatensystems angeordnet. Dadurch ist eine ortsaufgelöste Messung elektrischer Signale einer an dem Auflagebereich 5 angelagerten Zellpopulation möglich. Die Elektroden 6/7 können auch in anderer Weise in dem Auflagebereich 5 verteilt sein, beispielsweise in zueinander versetzten Reihen oder Spalten oder frei verteilt.

Auf und zwischen den Elektroden 6/7 können zur Optimierung des Wachstums der Zellen 3 Leitstrukturen angeordnet sein, die ein gezieltes Anlagern der Zellen 3 auch und/oder zwischen den Elektroden 6/7 ermöglichen. Die Leitstrukturen können beispielsweise eine Oberflächenstrukturierung, eine Beschichtung oder eine entsprechende Topographiegestaltung umfassen. Für unterschiedliche Zelltypen oder Meßaufgaben können verschiedene Abstände zwischen zueinander benachbarten aktiven Elektrodenbereichen 8 vorgesehen sein.

Insgesamt ergibt sich somit ein Verfahren zur Messung des Zellpotentials einer biologischen Zelle 3, bei dem die Zelle 3 in einem Nährmedium 2 adhärent an einem Auflagebereich 5 angelagert wird. Innerhalb des Auflagebereichs 5 der Zelle 3 wird mit Abstand von dessen Rand eine Öffnung in die Zellmembran der Zelle 3 eingebracht. Dabei dichtet der die Öffnung umgrenzende, an dem Auflagebereich 5 anhaftende Rand der Zellmembran die im Inneren der Zelle 3 befindliche Zellflüssigkeit gegen das Nährmedium 2 ab. Durch die Öffnung hindurch wird die elektrische Spannung zwischen Zellflüssigkeit und dem Nährmedium 2 gemessen.

Bei dem Ausführungsbeispiel nach Figur 19 ist zwischen der Meßelektrode 6 und dem Objektträger 4 ein Piezoelement 31 angeordnet, das an seinem freien, relativ zu dem Objektträger 4 beweglichen Ende die Meßelektrode 6 trägt und mit seinem dem freien Ende abgewandten Ende an dem Substrat 21 des Objektträgers 4 fixiert ist. Die Meßelektrode 6 ist wie bei dem Ausführungsbeispiel nach Figur 5 und 6 etwa kegelstumpfförmig ausgebildet und weist eine im wesentlichen zylindrische, sich entlang der Achse des Kegelstumpfs erstreckende Innenhöhlung 16 auf. Diese hat an dem dem Piezo-Element 31 abgewandten freien Ende der Meßelektrode 6 eine kreisrunde Öffnung, die von einem ringförmigen Elektrodenbereich umgrenzt ist, der eine von dem Auflagebereich 5 wegweisende scharfe Kante 32 aufweist. Mittels des Piezoelements 31 kann die Kante 32 der Meßelektrode 6 etwa in Richtung der Oberflächennormalen des Auflagebereichs 5 relativ zu dem Objektträger 4 auf eine an dem Auflagebereich 5 angelagerte Zelle 3 zu und von dieser wegbewegt werden. Dabei wird ein etwa kreisscheibenförmiger Bereich der Zellmembran aus dem Membranverbund der Zelle 3 herausgeschnitten, so daß die im Inneren der Zelle 3 befindliche Zellflüssigkeit durch die dabei entstehende Öffnung hindurch mit der Meßelektrode 6 in Kontakt gelangen kann. Die Innenhöhlung 16 der Meßelektrode 6 ist mit einem Elektrolyten gefüllt, durch den in der Zellflüssigkeit enthaltene Ionen nach dem Einbringen der Öffnung in die Zellmembran zu den die Innenhöhlung 16 begrenzenden Innenwänden der Meßelektrode 6 gelangen können. Dadurch wird ein guter elektrischer Kontakt zwischen der Zellflüssigkeit und der Meßelektrode 6 hergestellt.

In dem Auflagebereich 5 ist ein die Meßelektrode 6 umgrenzender elektrischer Isolator 9 angeordnet, der über die Kegelmantelfläche der Meßelektrode 6 bis dicht an deren scharfe Kante 32 herangeführt ist. Die an dem Auflagebereich 5 angelagerte Zelle 3 haftet mit ihrer Zellmembran an dem Isolator 9 an, wobei der die in die Zellmembran eingebrachte Öffnung umgrenzende Rand der Zellmembran die im Inneren der Zelle 3 befindliche Zellflüssigkeit gegen das Nährmedium 2 elektrisch isolierend abdichtet. Zum Abgreifen des Zellpotentials ist die Meßelektrode 6 mit einer in den Objektträger 4 integrierten Leiterbahn 18 mit einem Meßverstärker verbunden. Eine mit dem Nährmedium 2 in Kontakt befindliche Referenzelektrode 15 dient zur Ermittlung eines Referenzpotentials. Die Leiterbahn 18 und der Isolator 9 bestehen aus einem elastischen Material, das bei einer Ansteuerung des Piezoelements über die Steuerleitungen 42 eine Relativbewegung zwischen der Meßelektrode 6 und dem Substrat 21 des Objektträgers 4 ermöglicht.

Bei dem Ausführungsbeispiel nach Figur 20 ist zum Einbringen einer Öffnung in die Zellmembran einer an dem Auflagebereich 5 angelagerten Zelle 3 eine Laserdiode 33 vorgesehen, die von dem Auflagebereich 5 aus betrachtet hinter einem in der Meßelektrode 6 vorgesehenen optischen Fenster 34 angeordnet und in das Substrat 21 integriert ist. Die Laserdiode 33 ist mittels Anschlußleitungen 37 mit einer Stromversorgungs- und Steuereinrichtung verbunden. Das optische Fenster 34 ist als Durchgangslochung ausgebildet, welche die Meßelektrode 6 etwa in Richtung der Oberflächennormalen des Auflagebereichs 5 durchsetzt. Mittels der Laserdiode 33 kann ein Teilbereich der Zellmembran der an dem Auflagebereich 5 und der Meßelektrode 6 angelagerten biologischen Zelle 3 mit Laserstrahlung bestrahlt werden, welche die Zellmembran der Zelle 3 absorbiert. Dadurch wird eine Öffnung in die Zellmembran eingebracht. Nach dem Einbringen der Öffnung wird die Laserstrahlung abgeschaltet, so daß dann durch die Öffnung hindurch das Zellpotential mittels der Meßelektrode 6 gemessen werden kann. Wie bei dem Ausführungsbeispiel nach Figur 19 dichtet die an dem die Meßelektrode 6 umgrenzenden Isolator 9 anhaftende Zelle 3 die Meßelektrode 6 gegen das Nährmedium 2 ab.

Bei dem Ausführungsbeispiel gemäß Figur 21 ist zum Einbringen der Öffnung in die an dem Auflagebereich 5 anhaftende Zelle 3 ein externer Laser 35 vorgesehen, dessen Strahlung mittels einer Strahlführungseinrichtung 36, die zum Beispiel einen optischen Lichtleiter und/oder einen Umlenkspiegel sowie gegebenenfalls eine Fokusiereinrichtung umfassen kann, an der der Meßelektrode 6 abgewandten Rückseite des Objektträgers 4 durch ein in dem Substrat 21 vorgesehenes optisches Fenster und die die Meßelektrode 6 durchsetzende Lochung in die Zelle 3 eingekoppelt werden kann.

Bei dem Ausführungsbeispiel nach Figur 22 ist die Innenhöhlung 16 der Meßelektrode 6 mit einem Fluidkanal 38 verbunden, der mit einem steuerbaren Unterdruck beaufschlagbar ist. Der Fluidkanal 38 kann dazu beispielsweise an einer Mikropumpe angeschlossen sein, mittels der Nährmedium 2 aus dem Auflagebereich 5 des Objektträgers 4 abgesaugt werden kann. Dadurch wird das Anlagern einer Zelle 3 an der Meßelektrode 6 erleichtert. Gegebenenfalls kann nach dem Anlagern einer mittels des Fluidkanals 38 an die Meßelektrode 6 angesaugten Zelle noch für eine bestimmte Zeitdauer ein schwacher Unterdruck auf die Zelle 3 ausgeübt werden, bis diese selbständig an dem Auflagebereich 5 anhaftet.

Wie bei dem Ausführungsbeispiel nach Figur 19 weist der die Öffnung der Innenhöhlung 16 umgrenzende Rand der Meßelektrode 6 eine ringförmige scharfe Kante 32 auf, die gegenüber der Oberflächenebene des Auflagebereichs 5 vorsteht. Nach dem Anlagern der Zelle 3 an der Meßelektrode 6 wird ein Teilbereich der Zellmembran der Zelle 3 durch Absaugen von Nährmedium 2 an dem Fluidkanal 38 kurzzeitig so stark mit Unterdruck beaufschlagt, daß der von der scharfen Kante 32 der Meßelektrode 6 umgrenzte Membranbereich der Zellmembran aus dem Membranverbund herausgelöst wird. Dadurch wird eine Öffnung in die Zellmembran eingebracht, durch welche die Meßelektrode 6 mit der im Inneren der Zelle 3 befindlichen Zellflüssigkeit in Kontakt gelangen kann. Nach dem Einbringen der Öffnung wird der Unterdruck in dem Fluidkanal 38 abgeschaltet. Gegebenenfalls kann nach dem Einbringen der Öffnung noch eine geringe Menge Zellflüssigkeit aus dem Inneren der Zelle 3 in den Fluidkanal 38 angesaugt werden, so daß diese mit in dem Fluidkanal 38 benachbart zu der Meßelektrode 6 angeordneten Mikrosensoren 39 in Berührung gerät. Dadurch können zusätzliche Zellparameter, wie beispielsweise der Gasgehalt und/oder eine Ionenkonzentration der Zellflüssigkeit, gemessen werden.

Nach dem Einbringen der Öffnung in die Zellmembran der Zelle 3 kann die Förderrichtung der an den Fluidkanal 38 angeschlossenen Mikropumpe kurzzeitig umgekehrt werden, um eine in dem Fluidkanal 38 befindliche Substanz, beispielsweise ein Medikament und/oder einen Fluorenzfarbstoff durch die Öffnung der Zellmembran direkt in das Zellinnere zu injizieren. Wenn an der Elektrode 6 keine Zelle 3 angelagert ist, kann der Fluidkanal 38 außerdem dazu benutzt werden, um dem Nährmedium 2 eine entsprechende Substanz zuzugeben.

Bei dem Ausführungsbeispiel nach Figur 18 ist um das freie Ende der gegenüber der Oberflächenebene des Auflagebereichs 5 vorstehenden, als scharfe Spitze ausgebildeten Meßelektrode 6 herum in einem etwa ringförmigen Bereich eine chemische Substanz immobilisiert, die bei Berührung mit einer an dem Auflagebereich 5 angelagerten Zelle 3 eine Öffnung in deren Zellmembran einbringt. Durch diese Öffnung hindurch kann die Spitze der Meßelektrode 6 mit der Zellflüssigkeit der Zelle 3 in Berührung gelangen. Die Vorrichtung 1 weist einen besonders einfachen Aufbau auf.

## Patentansprüche

1. Verfahren zum Messen mindestens einer Zustandsgröße einer in einem Nährmedium (2) befindlichen, adhärent an einem Auflagebereich (5) angelagerten biologischen Zelle (3), wobei in die Zellmembran der Zelle (3) zum Messen der Zustandsgröße wenigstens eine Öffnung eingebracht wird, **dadurch gekennzeichnet, daß** die Öffnung innerhalb des Auflagebereichs (5) der Zelle (3) und mit Abstand von dessen Rand in die Zellmembran eingebracht wird und daß durch diese Öffnung hindurch die Zustandsgröße gemessen wird.

2. Verfahren nach Anspruch 1, **dadurch gekennzeichnet, daß** zum Messen des Zellpotentials der Zelle (3) durch die in die Zellmembran der Zelle (3) eingebrachte Öffnung hindurch die elektrische Spannung zwischen der Zellflüssigkeit und dem Nährmedium (2) gemessen wird.

3. Verfahren nach Anspruch 1 oder 2, **dadurch gekennzeichnet, daß** die Öffnung mittels Elektroporation in die Zellmembran eingebracht wird.

4. Verfahren nach einem der Ansprüche 1 bis 3, **dadurch gekennzeichnet, daß** die Zelle (3) durch eine Saugkraft an dem Auflagebereich (5) fixiert wird.

5. Verfahren nach einem der Ansprüche 1 bis 4, **dadurch gekennzeichnet, daß** nach dem Einbringen der Öffnung in die Zellmembran durch die Öffnung hindurch eine intrazelluläre Manipulation durchgeführt wird, insbesondere ein Medikament und/oder Fremdstoff und/oder eine biologische Substanz in das Innere der Zelle (3) gebracht wird.

6. Vorrichtung zur Messung mindestens einer Zustandsgröße wenigstens einer in einem Nährmedium (2) befindlichen biologischen Zelle (3), mit einem Objektträger (4), der einen Auflagebereich (5) hat, an dem die Zelle (3) adhärent anlagerbar ist, mit wenigstens einer mit der im Inneren der Zelle (3) befindlichen Zellflüssigkeit in Kontakt bringbaren Meßsonde zum Messen der Zustandsgröße, wobei die Meßsonde mit einem Meßverstärker verbunden oder verbindbar ist, **dadurch gekennzeichnet, daß** innerhalb des Auflagebereichs (5) die Meßsonde und ein diese umgrenzender elektrischer Isolator (9) angeordnet sind, derart, daß die Zelle (3) an dem Isolator (9) gegen das Nährmedium (2) dichtend anlagerbar ist, und daß zum Öffnen der Zellmembran der Zelle (3) im Bereich der Meßsonde zumindest ein Porationswerkzeug in dem Auflagebereich (5) angeordnet ist.

7. Vorrichtung nach Anspruch 6, **dadurch gekennzeichnet, daß** die Meßsonde eine Meßelektrode (6) ist, der zum Messen des Zellpotentials der Zelle (3) wenigstens eine mit dem Nährmedium (2) in Kontakt bringbare Referenzelektrode (15) zugeordnet ist.

8. Vorrichtung nach Anspruch 7, **dadurch gekennzeichnet, daß** die Meßelektrode (6) auch eine Elektroporations-Elektrode (7) ist, die zum Elektroporieren der Zellmembran der Zelle (3) mit einer elektrischen Spannungsquelle verbindbar ist, und daß die Elektroporations-Elektrode (6/7) wenigstens einen innerhalb des Auflagebereichs (5) angeordneten aktiven Elektrodenbereich (8) aufweist, der von dem elektrischen Isolator (9) umgrenzt ist.

9. Vorrichtung nach einem der Ansprüche 6 bis 8, **dadurch gekennzeichnet, daß** das Porationswerkzeug eine von der Meßsonde beabstandete Elektroporations-Elektrode (7) ist, die zum Elektroporieren der Zellmembran der Zelle (3) mit einer elektrischen Spannungsquelle verbindbar ist, daß die Elektroporations-Elektrode (6/7) wenigstens einen innerhalb des Auflagebereichs (5) angeordneten, von dem elektrischen Isolator (9) umgrenzten aktiven Elektrodenbereich (8) aufweist.

10. Vorrichtung nach einem der Ansprüche 6 bis 9, **dadurch gekennzeichnet, daß** das Porationswerkzeug zum Öffnen der Zellmembran der Zelle (3) mittels wenigstens eines Aktuators, insbesondere eines Piezoelements (31), quer zur Oberfläche des Auflagebereichs (5) relativ zu dem Objektträger (4) bewegbar ist.

11. Vorrichtung nach Anspruch 10, **dadurch gekennzeichnet, daß** der Aktuator mit einer Ansteuereinrichtung zum Erzeugen einer Ultraschallschwingung verbunden ist.

12. Vorrichtung nach einem der Ansprüche 6 bis 11, **dadurch gekennzeichnet, daß** der Objektträger (4) im Auflagebereich (5) eine Profilierung aufweist, die wenigstens eine um die Meßsonde umlaufende Profilierungsvertiefung (22) und/oder einen um die Meßsonde umlaufenden Profilierungsvorsprung hat.

13. Vorrichtung nach einem der Ansprüche 6 bis 12, **dadurch gekennzeichnet, daß** im Auflagebereich (5) des Objektträgers (4) an dessen Oberfläche eine wenigstens ein Zelladhäsionsprotein aufweisende Beschichtung und/oder eine hydrophile Beschichtung und/oder unmittelbar benachbart zu der Meßsonde eine hydrophobe Beschichtung angeordnet ist.

14. Vorrichtung nach einem der Ansprüche 6 bis 13, **dadurch gekennzeichnet, daß** in dem Auflagebereich mehrere Meßsonden vorzugsweise als Arrays angeordnet sind und daß diesen Meßsonden jeweils wenigstens ein Porationswerkzeug zugeordnet ist.

## Claims

1. Method of measuring at least one state variable of a biological cell (3) located in a nutrient medium (2) and settled in an adhering manner on a bearing area (5), wherein at least one opening is made in the cell membrane of the cell (3) in order to measure the state variable, **characterized in that** the opening is made in the cell membrane within the bearing area (5) of the cell (3) and at a distance from the edge of the latter, and **in that** the state variable is measured through this opening.

2. Method according to Claim 1, **characterized in that** in order to measure the cell potential of the cell (3) through the opening made in the cell membrane of the cell (3), the electrical voltage between the cell fluid and the nutrient medium (2) is measured.

3. Method according to Claim 1 or 2, **characterized in that** the opening is made in the cell membrane by means of electroporation.

4. Method according to any of Claims 1 to 3, **characterized in that** the cell (3) is fixed to the bearing area (5) by a suction force.

5. Method according to any of Claims 1 to 4, **characterized in that** once the opening has been made in the cell membrane an intracellular manipulation is carried out through the opening, in particular a medicament and/or foreign substance and/or a biological substance is introduced into the interior of the cell (3).

6. Device for measuring at least one state variable of at least one biological cell (3) located in a nutrient medium (2), said device comprising an object carrier (4) which has a bearing area (5) on which the cell (3) may settle in an adhering manner, and comprising at least one measurement probe for measuring the state variable which can be brought into contact with the cell fluid located in the interior of the cell (3), wherein the measurement probe is connected or can be connected to a measurement amplifier, **characterized in that** the measurement probe and an electrical insulator (9) that surrounds the latter are arranged within the bearing area (5) such that the cell (3) may settle on the insulator (9) in a sealed manner with respect to the nutrient medium (2), and **in that** at least one poration tool is arranged in the bearing area (5) in order to open the cell membrane of the cell (3) in the region of the measurement probe.

7. Device according to Claim 6, **characterized in that** the measurement probe is a measurement electrode (6) which in order to measure the cell potential of the cell (3) is assigned at least one reference electrode (15) which can be brought into contact with the nutrient medium (2).

8. Device according to Claim 7, **characterized in that** the measurement electrode (6) is also an electroporation electrode (7) which in order to electroporate the cell membrane of the cell (3) can be connected to an electrical power supply, and **in that** the electroporation electrode (6/7) has at least one active electrode area (8) arranged within the bearing area (5), said active electrode area being surrounded by the electrical insulator (9).

9. Device according to any of Claims 6 to 8, **characterized in that** the poration tool is an electroporation electrode (7) which is at a distance from the measurement probe and in order to electroporate the cell membrane of the cell (3) can be connected to an electrical power supply, and **in that** the electroporation electrode (6/7) has at least one active electrode area (8) which is arranged within the bearing area (5) and is surrounded by the electrical insulator (9).

10. Device according to any of Claims 6 to 9, **characterized in that** in order to open the cell membrane of the cell (3) the poration tool can be moved transversely to the surface of the bearing area (5) relative to the object carrier (4) by means of at least one actuator, in particular a piezo element (31).

11. Device according to Claim 10, **characterized in that** the actuator is connected to a control device in order to generate ultrasonic oscillation.

12. Device according to any of Claims 6 to 11, **characterized in that** the object carrier (4) in the bearing area (5) has a profiling which has at least one profiling depression (22) that surrounds the measurement probe and/or at least one profiling protrusion that surrounds the measurement probe.

13. Device according to any of Claims 6 to 12, **characterized in that** arranged in the bearing area (5) of the object carrier (4) on the surface of the latter there is a coating comprising at least one cell adhesion protein and/or a hydrophilic coating and/or a hydrophobic coating directly adjacent to the measurement probe.

14. Device according to any of Claims 6 to 13, **characterized in that** a number of measurement probes are arranged preferably as arrays in the bearing area and **in that** each of these measurement probes is assigned at least one poration tool.

## Revendications

1. Procédé de mesure d'au moins une grandeur d'état d'une cellule biologique (3) située dans un milieu de culture (2) et adhérente sur une zone de support (5), au moins une ouverture étant pratiquée dans la membrane cellulaire de la cellule (3) pour mesurer la grandeur d'état,
**caractérisé en ce que**
l'ouverture est pratiquée dans la membrane cellulaire à l'intérieur de la zone de support (5) de la cellule (3) et à distance du bord de cette zone, et la grandeur d'état est mesurée à travers cette ouverture.

2. Procédé selon la revendication 1,
**caractérisé en ce que**
pour mesurer le potentiel cellulaire de la cellule (3) on mesure à travers l'ouverture pratiquée dans la membrane cellulaire de la cellule (3) la tension électrique entre le liquide cellulaire et le milieu de culture (2).

3. Procédé selon la revendication 1 ou 2,
**caractérisé en ce que**
l'ouverture est pratiquée par électroporation dans la membrane cellulaire.

4. Procédé selon l'une quelconque des revendications 1 à 3,
**caractérisé en ce que**
la cellule (3) est fixée par une force d'aspiration à la zone de support (5).

5. Procédé selon l'une quelconque des revendications 1 à 4,
**caractérisé en ce qu'**
après la réalisation de l'ouverture dans la membrane cellulaire on effectue à travers l'ouverture une manipulation intracellulaire, notamment la mise en place à l'intérieur de la cellule (3) d'un médicament et/ou d'une matière étrangère et/ou d'une substance biologique.

6. Dispositif de mesure d'au moins une grandeur d'état d'une cellule biologique (3) située dans un milieu de culture (2), comportant un support d'objet (4) présentant une zone de support (5) sur laquelle la cellule (3) peut adhérer, avec au moins une sonde de mesure pouvant être mise en contact avec le liquide cellulaire se trouvant à l'intérieur de la cellule (3) pour mesurer la grandeur d'état, la sonde de mesure étant ou pouvant être reliée à un amplificateur de mesure,
**caractérisé en ce que**
la sonde de mesure et un isolateur électrique (9) entourant celle-ci sont disposés à l'intérieur de la zone de support (5), de telle sorte que la cellule (3) puisse être placée sur l'isolateur (9) de manière étanche par rapport au milieu de culture (2), et pour ouvrir la membrane cellulaire de la cellule (3) dans la zone de la sonde de mesure au moins un outil de poration est disposé dans la zone de support (5).

7. Dispositif selon la revendication 6,
**caractérisé en ce que**
la sonde de mesure est une électrode de mesure (6) à laquelle est associée, pour mesurer le potentiel cellulaire de la cellule (3), au moins une électrode de référence (15) pouvant être mise en contact avec le milieu de culture (2).

8. Dispositif selon la revendication 7,
**caractérisé en ce que**
l'électrode de mesure (6) est également une électrode d'électroporation (7), qui pour l'électroporation de la membrane cellulaire de la cellule (3) peut être reliée à une source de tension électrique, et l'électrode d'électroporation (6/7) présente au moins une zone d'électrode active (8) disposée à l'intérieur de la zone de support (5) et entourée par l'isolateur électrique (9).

9. Dispositif selon l'une quelconque des revendications 6 à 8,
**caractérisé en ce que**
l'outil de perforation est une électrode d'électroporation (7) distancée de la sonde de mesure, qui pour l'électroporation de la membrane cellulaire de la cellule (3) peut être reliée à une source de tension électrique, et l'électrode d'électroporation (6/7) présente au moins une zone d'électrode active (8) disposée à l'intérieur de la zone de support (5) et entourée par l'isolateur électrique (9).

10. Dispositif selon l'une quelconque des revendications 6 à 9,
**caractérisé en ce que**
l'outil de perforation, pour ouvrir la membrane cellulaire de la cellule (3) au moyen d'au moins un actionneur, notamment un piézo-élément (31), peut être déplacé par rapport au support d'objet (4) transversalement à la surface de la zone de support (5).

11. Dispositif selon la revendication 10,
**caractérisé en ce que**
l'actionneur est relié à une installation de commande pour générer une oscillation ultrasonore.

12. Dispositif selon l'une quelconque des revendications 6 à 11,
**caractérisé en ce que**
dans la zone de support (5) le support d'objet (4) présente un profilage qui est pourvu d'au moins un creux de profilage (22) s'étendant autour de la sonde de mesure et/ou d'une saillie de profilage s'étendant autour de la sonde de mesure.

13. Dispositif selon l'une quelconque des revendications 6 à 12,
**caractérisé en ce que**
dans la zone de support (5) du support d'objet (4), à la surface de celle-ci, est disposé un revêtement présentant une protéine d'adhésion cellulaire et/ou un revêtement hydrophile et/ou, à proximité immédiate de la sonde de mesure, un revêtement hydrophobe.

14. Dispositif selon l'une quelconque des revendications 6 à 13,
**caractérisé en ce que**
dans la zone de support plusieurs sondes de mesure sont disposées, de préférence en rangées, et au moins un outil de poration est chaque fois associé à ces sondes de mesures.
